# EUROPÄISCHE PATENTSCHRIFT

(11) **EP 3 840 700 B1**
(45) Veröffentlichungstag und Bekanntmachung des Hinweises auf die Patenterteilung: **14.10.2026**
(21) Anmeldenummer: 19758657.1
(22) Anmeldetag: 17.08.2019
(51) Int. Cl.: A61F 5/01, B25J 9/00, A61H 1/02

(54) **SYSTEM UND VERFAHREN ZUR REDUKTION VON AUF EINEN ARM EINES MENSCHEN WIRKENDEN KRÄFTEN**
SYSTEM AND METHOD FOR THE REDUCTION OF FORCES ACTING ON AN ARM OF A HUMAN
SYSTÈME ET PROCÉDÉ POUR RÉDUIRE LES FORCES AGISSANT SUR LE BRAS D'UN ÊTRE HUMAIN

(30) Priorität: 22.08.2018 DE 102018120453
(43) Veröffentlichungstag der Anmeldung: 30.06.2021
(73) Patentinhaber: exoIQ GmbH, 21109 Hamburg (DE)
(72) Erfinder: WEIDNER, Robert, 29581 Gerdau (DE); OTTEN, Bernward, 20535 Hamburg (DE)
(74) Vertreter: Koplin, Moritz
(86) Internationale Anmeldenummer: PCT/EP2019/072069
(87) Internationale Veröffentlichungsnummer: WO 2020/038850

(56) Entgegenhaltungen:
- EP-A1- 1 609 451
- EP-A1- 3 156 193
- EP-A1- 3 504 031
- EP-B1- 3 504 031
- WO-A1-2018/065459
- WO-A2-2013/186705
- DE-A1- 102016 121 203
- DE-A1- 102017 112 436
- JP-A- 2017 024 086
- JP-A- 2017 024 086
- KR-A- 20160 104 954
- KR-A- 20160 104 954

## Beschreibung

### TECHNISCHES GEBIET

Die vorliegende Erfindung bezieht sich auf Exoskelette. Insbesondere bezieht sich die vorliegende Erfindung auf Exoskelette, die zur Stützung der Arme eingerichtet sind.

### STAND DER TECHNIK

Exoskelette werden typischerweise auf natürliche Bewegungsmuster abgestimmt. Dabei ist in Hinblick auf die Stützung der Arme das Schultergelenk von zentraler Bedeutung. Die Hauptfunktion des Schultergelenks wird durch die Rotation des Oberarmknochen (Humerus) im Schulterdach (Akromiom) realisiert. Diese Rotation erlaubt insgesamt drei Freiheitsgrade. Darüber hinaus ist das Schulterblatt über das Schlüsselbein (Klavikula) gelenkig an das Brustbein (Sternum) gekoppelt und gibt dem Schultergürtel zwei weitere Freiheitsgrade (Auf-/Abwärtsbewegung sowie Vor- und Zurückbewegung des Schultergürtels). Die hohe Komplexität des Schultergürtels macht es schwierig, die daraus resultierenden Bewegungsmuster mit geringem technischen Aufwand nachzubilden.

Ein Ansatz zur Nachbildung basiert auf Scharnierkonstruktionen, bei denen zwei Elemente seriell über eine vertikale Achse gekoppelt sind. Das erste Element kann bspw. an eine Stützstruktur (bspw. einen Rucksack) gekoppelt sein und das zweite Ende kann sich frei in einer Ebene parallel zum Boden bewegen (vgl. bspw. die Druckschrift EP 3189945 A1). Am freien Ende können unterschiedliche Aktoren angebracht sein, die ein Drehmoment um eine horizontale Achse erzeugen. An dem angetriebenen Ende dieser horizontalen Achse kann wiederum eine Befestigungsmöglichkeit und/oder eine Auflagefläche für den Oberarm angeordnet sein. Die Stützstruktur und die Verbindungselemente sind dabei typischerweise so gestaltet, dass die angetriebene horizontale Achse möglichst den Drehpunkt von Oberarmknochen und Schulterdach trifft.

Ein anderer Ansatz, der beispielsweise in der US 2012/184880 A1 gezeigt ist, basiert auf der Positionierung eines Drehgelenks über der Schulter, wobei das Drehgelenk eine vertikale Drehachse aufweist, die näherungsweise durch das Schultergelenk (Glenohumeralgelenk) verläuft. Von diesem Drehgelenk verläuft ein Verbindungselement zu einer angetriebenen horizontalen Achse. Im Gegensatz zum zuvor beschriebenen Ansatz ist hier keine stramme Anbindung an den Arm des Nutzers erforderlich, da sich die angetriebene Achse nicht frei in einer zum Boden parallelen Ebene bewegen kann, sondern lediglich um die vertikale Achse. Die JP 2017 024086 A offenbart eine Vorrichtung gemäß dem Oberbegriff des Anspruchs 1.

### ZUSAMMENFASSUNG DER ERFINDUNG

Die Erfindung ist in den beigefügten Ansprüchen wiedergegeben.

### KURZBESCHREIBUNG DER ERFINDUNG

Erfindungsgemäß werden Kräfte, die am Arm in vertikaler Richtung angreifen, (im Wesentlichen) über eine mehrgliedrige Gelenkkette in die Stützstruktur eingeleitet. Im Unterschied zu dem oben zuallererst beschriebenen Ansatz werden die verbleibenden Kräfte jedoch nicht über den Arm des Nutzers abgeleitet. Vielmehr wird die Bewegung der Gelenkkette über eine zweite über der Schulter verlaufende flexible Struktur eingeschränkt (bzw. geführt). Diese flexible Struktur kann bspw. aus zwei Elementen bestehen, die (analog zum zweiten oben beschriebenen Ansatz) mittels eines Gelenks, das über der Schulter angeordnet ist, miteinander verbunden sind. Durch die Verformbarkeit der flexiblen Struktur kann das System kompakter ausgeführt werden, da die flexible Struktur unmittelbar über der Schulter angeordnet werden kann, ohne eine aus einem Heben der Hand des Nutzers resultierenden Anhebung der Schulter zu behindern.

Ein erfindungsgemäßes System zur Reduktion von auf einen Arm eines Nutzers wirkenden Kräften umfasst ein Beckenelement zur Anbindung an das Becken des Nutzers, ein Schulterelement zur Anbindung an den Schultergürtel des Nutzers, ein Wirbelsäulenelement, welches das Schulterelement mit dem Beckenelement verbindet und die auf das Schulterelement wirkenden Kräfte auf das Beckenelement ableitet, und eine Anordnung zur Stützung des Arms des Nutzers. Die Anordnung weist einen Aktor auf, der durch eine Gelenkkette mit dem Schulterelement verbunden ist, welche sich entlang des Schulterblatts einer Schulter des Nutzers erstreckt. Die Gelenkkette leitet die in vertikaler Richtung wirkenden Kräfte in das Schulterelement ein und wird mittels einer flexiblen Struktur, welche sich über der Schulter des Nutzers erstreckt, geführt.

Dabei ist unter dem Begriff "Beckenelement", wie er in der Beschreibung und den Ansprüchen verwendet wird, insbesondere eine (teilweise flexible, bspw. textile) Struktur zu verstehen, die während der Verwendung des Systems das Becken des Nutzers umgreift oder umschließt. Ferner ist unter dem Begriff "Schulterelement", wie er in der Beschreibung und den Ansprüchen verwendet wird, insbesondere eine (teilweise flexible, bspw. textile) Struktur zu verstehen, die während der Verwendung des Systems die Schultern des Nutzers umgreift oder umschließt. Des Weiteren ist unter dem Begriff "Wirbelsäulenelement", wie er in der Beschreibung und den Ansprüchen verwendet wird, insbesondere eine aus mehreren rigiden oder flexiblen Elementen bestehende, aktive oder passive Verbindungsstruktur zu verstehen, die eine Kraftübertragung zwischen dem Schulterelement und dem Beckenelement ermöglicht.

Zudem ist unter der Formulierung "welche sich entlang des Schulterblatts einer Schulter des Nutzers erstreckt", wie sie in der Beschreibung und den Ansprüchen verwendet wird, insbesondere eine Gelenkkette zu verstehen, die auf Höhe des Schulterblatts (im Wesentlichen horizontal) vom Rücken zu einer Körperseite verläuft. Des Weiteren ist unter der Formulierung "welche sich über der Schulter des Nutzers erstreckt", wie sie in der Beschreibung und den Ansprüchen verwendet wird, insbesondere eine flexible Struktur zu verstehen, die vertikal über dem Schultergelenk angeordnet ist (und bspw. auf der Schulter aufliegt).

Vorzugsweise ist die Gelenkkette eingerichtet, eine Bewegung des Aktors in einer horizontalen Ebene zu ermöglichen und eine Bewegung des Aktors in vertikaler Richtung zu verhindern. Bspw. können die Gelenke der Gelenkkette parallele (vertikale) Drehachsen aufweisen.

Dabei ist anzumerken, dass die Begriffe "horizontal" und "vertikal", wie sie in der Beschreibung und den Ansprüchen verwendet werden, sich auf ein Szenario beziehen, in dem der Nutzer aufrecht steht.

Vorzugsweise weist die flexible Struktur miteinander gelenkig verbundene Elemente auf.

Bspw. können die gelenkig miteinander verbundenen Elemente relativ zueinander um eine Drehachse drehbar sein. Die Elemente können dabei so miteinander verbunden sein, dass sich die Drehachse in vertikaler Richtung erstreckt. Insbesondere kann die flexible Struktur eingerichtet sein, den Aktor auf einer in einer horizontalen Ebene liegenden Kreisbahn zu führen. Vorzugsweise liegt dabei der Drehpunkt des Schultergelenks im Mittelpunkt der Kreisbahn.

Vorzugsweise weist eines der miteinander gelenkig verbundenen Elemente einen gekrümmten Abschnitt auf, der dazu vorgesehen ist, eine vertikale Verschiebung eines Teils der flexiblen Struktur mittels eines vertikalen Versatzes zwischen den Enden des gekrümmten Abschnitts auszugleichen. Bspw. kann der der gekrümmte Abschnitt L- oder U-förmig sein.

Das System kann in einem Verfahren zur Reduktion von auf einen Arm eines Nutzers wirkenden Kräften verwendet werden, welches ein Anbinden des Systems an das Becken und den Schultergürtel des Nutzers und ein Steuern des Aktors zur Reduktion der auf den Arm des Menschen wirkenden Kräfte umfasst, wobei eine Behinderung einer aus einem Heben einer Hand des Nutzers resultierenden Anhebung der Schulter durch die Verformbarkeit der flexiblen Struktur vermieden wird.

Dabei versteht es sich, dass alle Merkmale (bevorzugter Ausführungsformen) des erfindungsgemäßen Systems auch Merkmale (bevorzugter Ausführungsformen) des erfindungsgemäßen Verfahrens sein können, welches sich auf eine (mögliche) Verwendung des erfindungsgemäßen Systems bezieht.

### KURZE BESCHREIBUNG DER ZEICHNUNGSFIGUREN

Die Erfindung wird nachfolgend in der detaillierten Beschreibung anhand von Ausführungsbeispielen erläutert, wobei auf Zeichnungsfiguren Bezug genommen wird, in denen:
Fig. 1 eine schematische perspektivische Ansicht eines ersten erfindungsgemäßen Systems;
Fig. 2 bis Fig. 4 schematische perspektivische Ansichten eines zweiten erfindungsgemäßen Systems;
Fig. 5 bis Fig. 7 schematische perspektivische Ansichten eines dritten erfindungsgemäßen Systems; und
Fig. 8 ein Ablaufdiagramm eines Verfahrens zur Reduktion von auf einen Arm eines Nutzers wirkenden Kräften zeigt.

Dabei versteht es sich, dass die gezeigten Elemente nicht notwendigerweise maßstabs- und detailgetreu dargestellt sind, sondern primär der Veranschaulichung der erfinderischen Idee dienen.

### BESCHREIBUNG DER AUSFÜHRUNGSARTEN

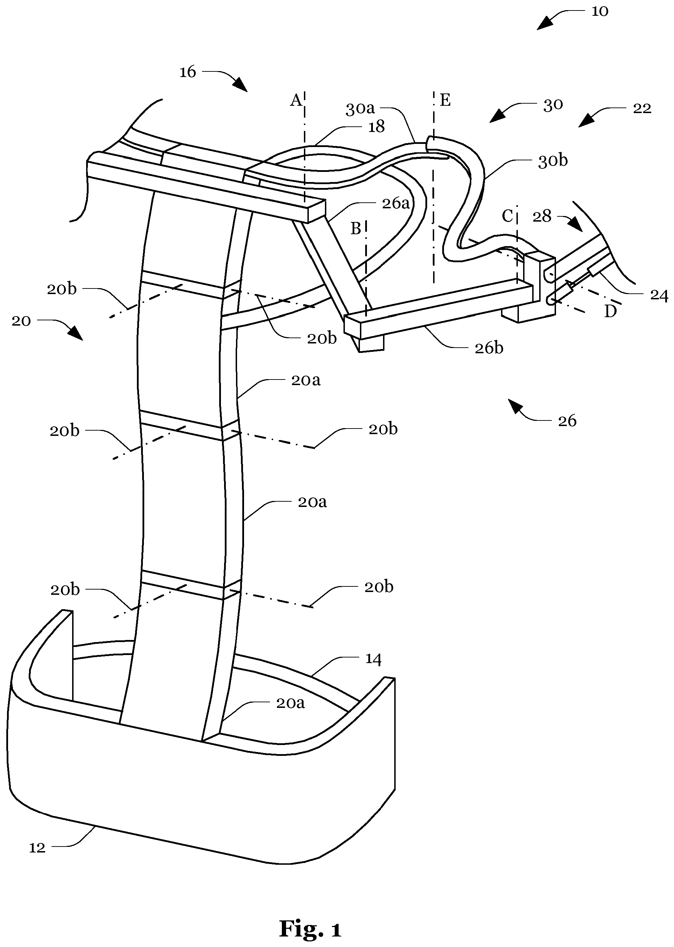

Fig. 1 zeigt ein System 10 zur Reduktion von auf einen Arm eines Nutzers wirkenden Kräften. Das System 10 umfasst ein Beckenelement 12 zur Anbindung an das Becken des Nutzers. Beim Anlegen des Systems 10 wird das Beckenelement 12 so angeordnet, dass es das Becken des Benutzers umgreift und am Körper des Nutzers anliegt, wodurch eine Kraftübertragung zwischen dem System 10 und dem Rumpf des Nutzers ermöglicht wird. Das Beckenelement 12 kann ferner zur Sicherung der Kraftübertragung mit einem Beckengurt 14 versehen oder in ein (textiles) Kleidungsstücke integriert sein, welcher/welches das Becken des Nutzers umschließt.

Das System 10 umfasst ferner ein Schulterelement 16 zur Anbindung an den Schultergürtel des Nutzers. Beim Anlegen des Systems 10 wird das Schulterelement 16 so angeordnet, dass es die Schultern des Benutzers umgreift und am Körper des Nutzers anliegt, wodurch eine Kraftübertragung zwischen dem System 10 und dem Oberkörper des Nutzers ermöglicht wird. Das Schulterelement 16 kann ferner zur Sicherung der Kraftübertragung mit Schultergurten 18 versehen (in Fig. 1 exemplarisch nur für die rechte Schulter gezeigt) oder in ein (textiles) Kleidungsstücke integriert sein, welche/welches die Schultern des Nutzers (teilweise oder ganz) umschließen/umschließt.

Das Schulterelement 16 ist mit dem Beckenelement 12 über ein Wirbelsäulenelement 20 verbunden, welches die auf das Schulterelement 16 wirkenden Kräfte auf das Beckenelement 12 ableitet. Das Wirbelsäulenelement 20 besteht aus mehreren rigiden Segmenten 20a, die relativ zueinander um zwei (im aufrechten Zustand im Wesentlichen) horizontale Drehachsen 20b drehbar sind, wodurch ein Beugen des Oberkörpers ermöglicht wird. Zur Unterstützung des Aufrichtens des Oberkörpers kann das Wirbelsäulenelement 20 mit einem oder mehrerer Aktoren (nicht gezeigt) versehen sein, mittels derer die Relativpositionen der Segmente 20a zueinander steuerbar ist.

Jedoch versteht es sich, dass die in Fig. 1 gezeigte Ausgestaltung des Wirbelsäulenelements 20 nur eine beispielhafte Möglichkeit ist und die Erfindung diesbezüglich nicht beschränkt ist. Bspw. können anstatt der rigiden Segmente 20a auch flexible Segmente 20a eingesetzt werden, deren Steifheit mittels Aktoren eingestellt wird, und die relative Verdrehung der Segmente 20a mit einer relativen Verschiebung der Segmente 20a überlagert werden. Zudem kann das Wirbelsäulenelement 20 mehr oder weniger Segmente 20a umfassen, als in Fig. 1 gezeigt.

Wie in Fig. 1 gezeigt, ist an das Schulterelement 16 eine Anordnung 22 zur Stützung des Arms des Nutzers angekoppelt. Die Anordnung 22 weist einen Aktor 24 in Form eines Pneumatikzylinders auf, der durch eine zweigliedrige Gelenkkette 26 mit dem Schulterelement 16 verbunden ist. Die Gelenkkette 26 umfasst ein erstes Element 26a, wobei ein erstes Ende des ersten Elements 26a um eine vertikale Achse A drehbar an das Schulterelement 16 angekoppelt ist, und ein zweites Element 26b, wobei ein erstes Ende des zweiten Elements 26b um eine vertikale Achse B drehbar an das zweite Ende des ersten Elements 26a angekoppelt ist. Wie in Fig. 1 gezeigt, ist der Aktor 24 um eine vertikale Achse C drehbar an das zweite Ende des zweiten Elements 26b angekoppelt und bewirkt eine Drehbewegung einer Oberarm-, Unterarm- oder Handgelenkstütze 26 um eine horizontale Achse D.

Die Gelenkkette 26 wird zudem mittels einer flexiblen Struktur 30 geführt, die die Schulter des Nutzers überspannt (bzw. auf der Schulter des Nutzers aufliegt). Die flexible Struktur 30 ist in vertikaler Richtung biegeweich und überträgt somit (so gut wie) keine Kräfte in vertikaler Richtung. Wie in Fig. 1 gezeigt, kann die flexible Struktur 30 zwei Elemente 30a, 30b aufweisen, die miteinander (um eine sich in vertikaler Richtung erstreckende Drehachse E drehbar) gelenkig verbunden sind. Ist die Armstütze 26 als Oberarmstütze ausgebildet und schneiden sich die Drehachsen D und E im Drehpunkt des Schultergelenks, kann der Oberarm in seinem natürlichen Bewegungsmuster unterstützt werden.

Um eine aus einem Heben einer Hand des Nutzers resultierende Anhebung der Schulter möglichst wenig zu behindern, weist eines der miteinander gelenkig verbundenen Elemente 30a, 30b einen gekrümmten, U-förmigen Abschnitt auf, der dazu vorgesehen ist, eine vertikale Verschiebung eines Teils der flexiblen Struktur 30 mittels eines vertikalen Versatzes zwischen den Enden des gekrümmten Abschnitts auszugleichen. Anders ausgedrückt ist das zweite Element 30b der flexiblen Struktur 30 zwischen dem Aktor 24 und dem Drehpunkt über der Schulter in der Form zweier "L" auf einem nicht direkten Wege geführt. Dies erleichtert das Anheben der Schulter, erlaubt jedoch eine sichere Führung des Aktors 24 in einer Kreisbahn um den Schulterdrehpunkt. Ein ähnlicher indirekter Kraftfluss kann zudem für das erste Element 30a der flexiblen Struktur 30 vorgesehen sein.

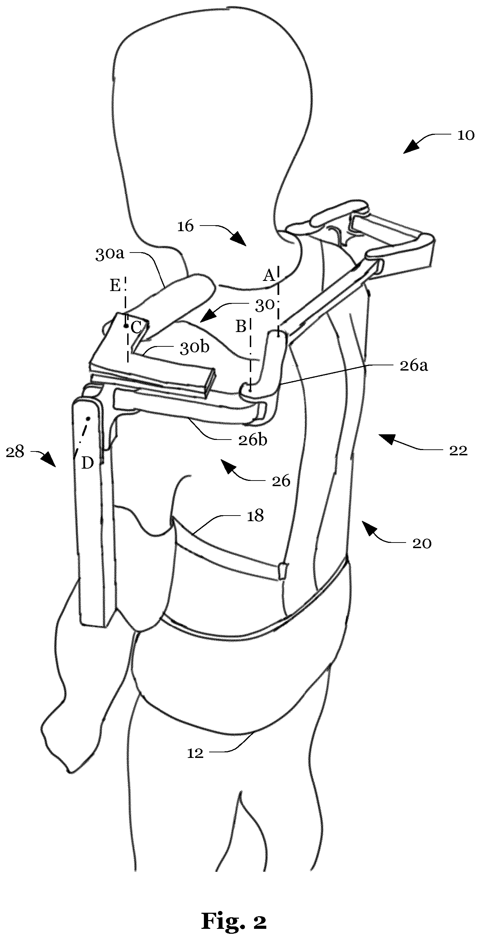

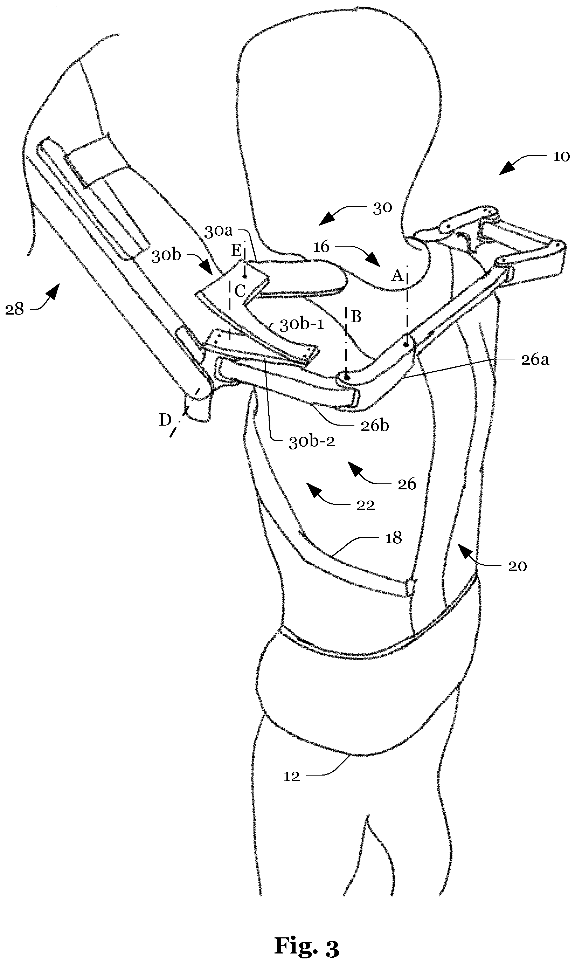

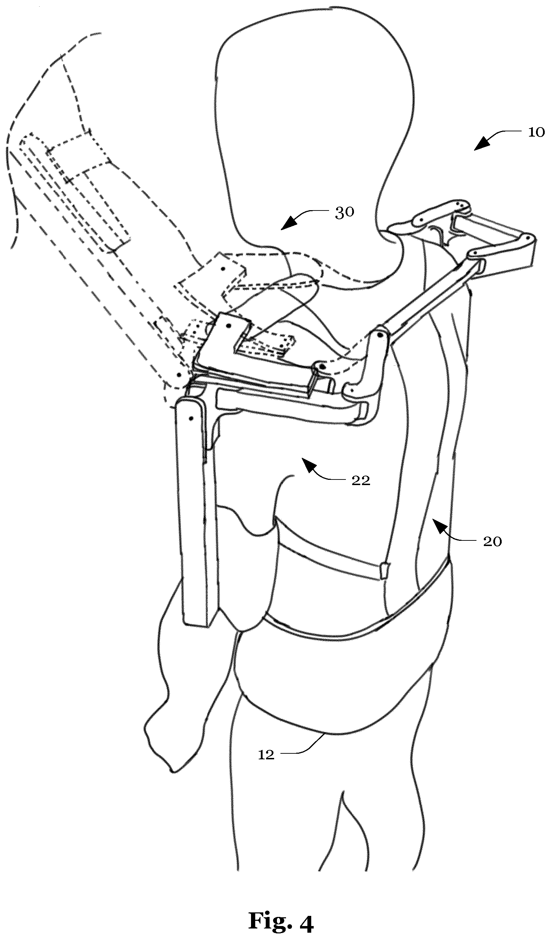

Fig. 2 zeigt eine weitere mögliche Ausgestaltung eines erfindungsgemäßen Systems 10, welches sich von dem in Fig. 1 gezeigten System 10 dadurch unterscheidet, dass das zweite Element 30b der flexiblen Struktur einen L-förmigen Abschnitt 30b-1 aufweist, dessen distales Ende mittels eines Verbindungsstücks 30b-2 mit der Gelenkkette 26 verbunden ist. Wie in Fig. 3 gezeigt, ist das Material des zweiten Elements 30b so gewählt, dass sich beim Heben der Schulter der ursprünglich flache L-förmige Abschnitt 30b-1 von dem ursprünglich flachen Verbindungsstück 30b-2 in vertikaler Richtung wegbiegen lässt (wobei sich der L-förmige Abschnitt verdreht). Wie in Fig. 4 veranschaulicht, gibt somit beim Heben des Armes die flexible Struktur 30 nach, so dass zwischen der flexiblen Struktur 30 und der Schulter (bei gesenktem Arm) kein Abstand vorgesehen sein muss und das System 10 kompakter ausgelegt werden kann.

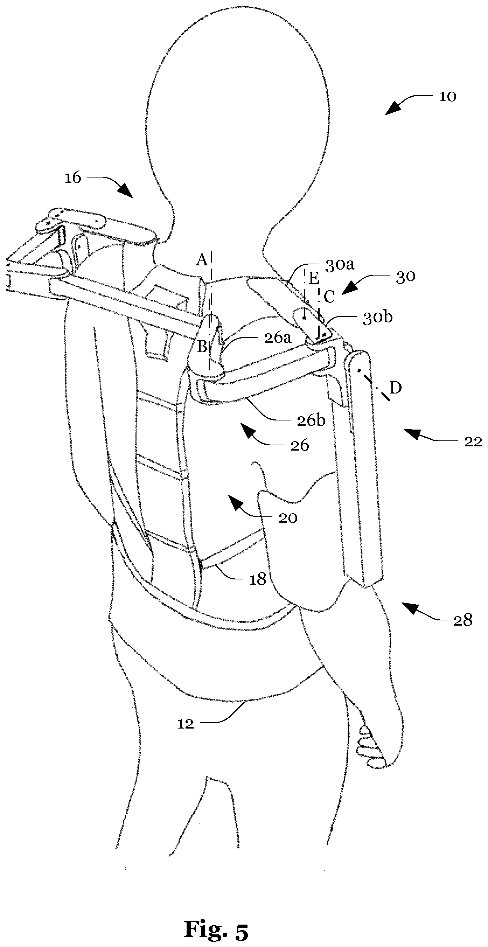

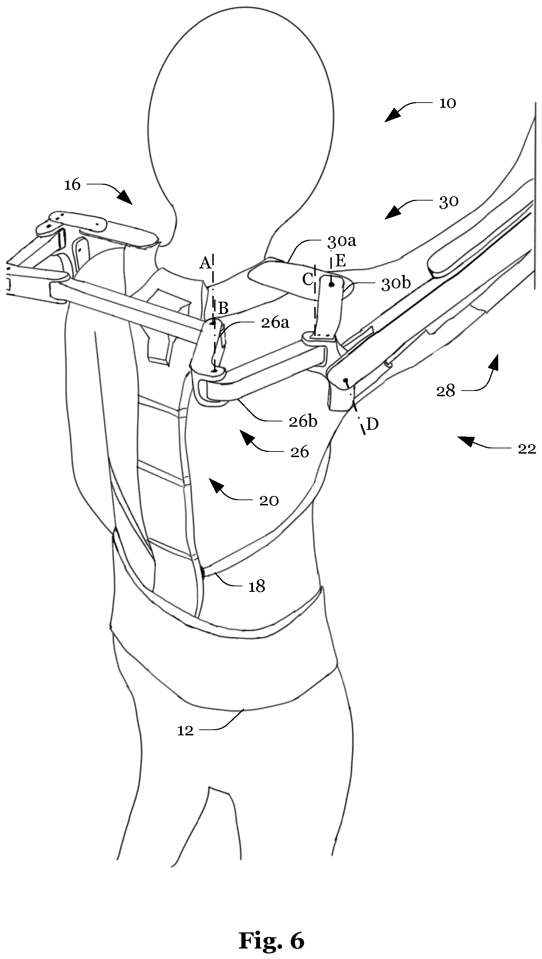

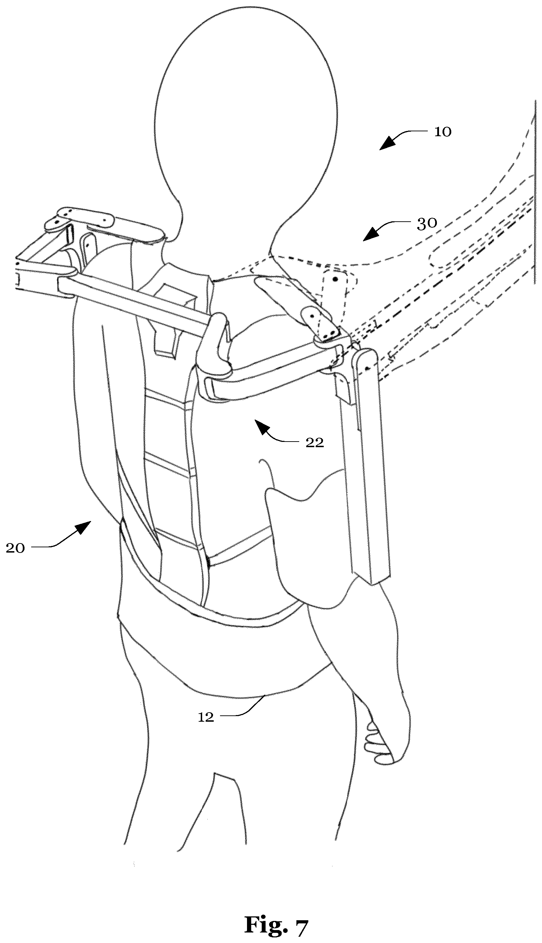

Fig. 5 bis Fig. 7 zeigen eine weitere mögliche Ausgestaltung eines erfindungsgemäßen Systems 10, welches sich von dem in Fig. 2 bis Fig. 4 gezeigten System 10 dadurch unterscheidet, dass das zweite Element 30b als flexibles (textiles) Band ausgebildet ist. Wie in Fig. 6 gezeigt, ist das Material des flexiblen Bands so gewählt, dass sich dieses beim Heben der Schulter dehnen und den dadurch vergrößerten Höhenversatz ausgleichen kann. Wie in Fig. 7 veranschaulicht, wird somit beim Heben des Armes das Anheben der Schulter des Benutzers nicht behindert, da sich das flexible Band zwar über die Schulter spannt, die Schulterbewegung aber nicht blockiert.

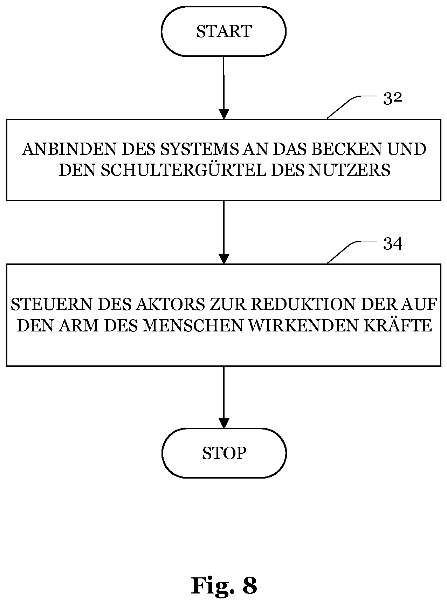

Fig. 8 zeigt ein Verfahren zur Reduktion der auf einen Arm eines Nutzers wirkenden Kräfte. Dabei wird bei Schritt 32 das Beckenelement 12 an das Becken und das Schulterelement 16 an die Schultern angebunden und bei Schritt 34 der Aktor 24 zur Reduktion der auf den Arm des Menschen wirkenden Kräfte gesteuert. Bspw. kann das System 10 mit Sensoren versehen sein, aus deren Messwerten ein Belastungszustand des Nutzers abgeleitet werden kann und der Nutzer entsprechend seines Belastungszustands unterstützt werden. Insbesondere kann der Benutzer bei einer Tätigkeit unterstützt werden, bei der der Benutzer die Hände oberhalb seines Kopfes hält, um einer Ermüdung des Nutzers vorzubeugen oder eine ansonsten für den Nutzer nicht bewältigbare Kraftanstrengung zu reduzieren.

### LISTE DER BEZUGSZEICHEN

- 10: System
- 12: Beckenelement
- 14: Beckengurt
- 16: Schulterelement
- 18: Schultergurt
- 20: Wirbelsäulenelement
- 20a: Segment
- 20b: Drehachse
- 22: Anordnung
- 24: Aktor
- 26: Gelenkkette
- 26a: Element (Gelenkkette)
- 26b: Element (Gelenkkette)
- 28: (Arm-)Stütze
- 30: flexible Struktur
- 30a: Element
- 30b: Element
- 30b-1: L-förmiger Abschnitt
- 30b-2: Verbindungsstück
- 32: Prozessschritt
- 34: Prozessschritt
- A-E: Drehachsen

## Patentansprüche

1. System (10) zur Reduktion von auf einen Arm eines Nutzers wirkenden Kräften, umfassend:
ein Beckenelement (12), eingerichtet zur Anbindung an das Becken des Nutzers; ein Schulterelement (16), eingerichtet zur Anbindung an den Schultergürtel des Nutzers;
ein Wirbelsäulenelement (20), welches das Schulterelement (16) mit dem Beckenelement (12) verbindet und zur Ableitung von auf das Schulterelement (16) wirkenden Kräften auf das Beckenelement (12) eingerichtet ist; und
eine Anordnung (22) zur Stützung des Arms des Nutzers;
wobei die Anordnung (22) einen Aktor (24) aufweist, der durch eine Gelenkkette (26) mit dem Schulterelement (16) verbunden ist, und die Gelenkkette (26) eingerichtet ist, sich entlang des Schulterblatts einer Schulter des Nutzers zu erstrecken und in vertikaler Richtung wirkende Kräfte in das Schulterelement (16) einzuleiten;
**dadurch gekennzeichnet, dass**
die Gelenkkette (26) mittels einer flexiblen Struktur (30), welche eingerichtet ist, sich über der Schulter des Nutzers zu erstrecken, geführt wird.

2. System (10) nach Anspruch 1, wobei die Gelenkkette (26) eingerichtet ist, eine Bewegung des Aktors (24) in einer horizontalen Ebene zu ermöglichen und eine Bewegung des Aktors (24) in vertikaler Richtung zu verhindern, wenn der Nutzer aufrecht steht.

3. System (10) nach Anspruch 2, wobei die Gelenke der Gelenkkette (26) parallele Drehachsen (A-C) aufweisen.

4. System (10) nach Anspruch 3, wobei sich die parallelen Drehachsen (A-C) in vertikaler Richtung erstrecken, wenn der Nutzer aufrecht steht.

5. System (10) nach einem der Ansprüche 1 bis 4, wobei die Gelenkkette (26) als ebene Drehgelenkkette ausgebildet ist.

6. System (10) nach einem der Ansprüche 1 bis 5, wobei die flexible Struktur (30) eines oder mehrere längliche und/oder flache Elemente (30a, 30b) umfasst, die eingerichtet sind, entgegen einer Zugrichtung eine Führungskraft in die Gelenkkette (26) einzuleiten.

7. System (10) nach Anspruch 6, wobei das eine bzw. die mehreren länglichen und/oder flachen Elemente (30a, 30b) quer zur Zugrichtung biegbar oder biegeweich sind.

8. System (10) nach einem der Ansprüche 1 bis 7, wobei die flexible Struktur (30) miteinander gelenkig verbundene Elemente (30a, 30b) aufweist.

9. System (10) nach Anspruch 8, wobei sich eine Drehachse (E) der miteinander gelenkig verbundenen Elemente (30a, 30b) in vertikaler Richtung erstreckt, wenn der Nutzer aufrecht steht.

10. System (10) nach Anspruch 9, wobei die flexible Struktur (30) eingerichtet ist, den Aktor (24) auf einer in einer horizontalen Ebene liegenden Kreisbahn zu führen, wenn der Nutzer aufrecht steht.

11. System (10) nach Anspruch 10, wobei der Drehpunkt des Schultergelenks des Nutzers im Mittelpunkt der Kreisbahn liegt.

12. System (10) nach einem der Ansprüche 8 bis 11, wobei eines der miteinander gelenkig verbundenen Elemente (30a, 30b) einen gekrümmten Abschnitt aufweist, der dazu vorgesehen ist, eine vertikale Verschiebung eines Teils der flexiblen Struktur (30) mittels eines vertikalen Versatzes zwischen den Enden des gekrümmten Abschnitts auszugleichen.

13. System (10) nach Anspruch 12, wobei der gekrümmte Abschnitt L- oder U-förmig ist.

14. Verfahren zur Reduktion von auf einen Arm eines Nutzers wirkenden Kräften, umfassend:
Anbinden (32) eines Systems (10) nach einem der Ansprüche 1 bis 13 an das Becken und den Schultergürtel des Nutzers; und
Steuern (34) des Aktors (24) zur Reduktion der auf den Arm des Menschen wirkenden Kräfte;
wobei eine Behinderung einer aus einem Heben einer Hand des Nutzers resultierenden Anhebung der Schulter durch die Verformbarkeit der flexiblen Struktur (30) vermieden wird.

## Claims

1. A system (10) for reducing forces acting on a user's arm, comprising:
a pelvic element (12) which is configured to be attached to the user's pelvis;
a shoulder element (16) which is configured to be attached to the user's shoulder girdle;
a spinal element (20) which connects the shoulder element (16) to the pelvic element (12) and which is configured to transmit forces acting on the shoulder element (16) to the pelvic element (12); and
an arrangement (22) for supporting the user's arm;
wherein the arrangement (22) comprises an actuator (24) connected to the shoulder element (16) by a kinematic chain (26), the kinematic chain (26) being configured to extend along the scapula of a user's shoulder and to transmit forces acting in a vertical direction onto the shoulder element (16);
**characterized in that**
the kinematic chain chain (26) is guided by means of a flexible structure (30) which is configured to extend over the user's shoulder.

2. The system (10) of claim 1, wherein the kinematic chain (26) is configured to permit movement of the actuator (24) in a horizontal plane and to prevent movement of the actuator (24) in a vertical direction when the user stands upright.

3. The system (10) of claim 2, wherein the joints of the kinematic chain (26) comprise parallel axes of rotation (A-C).

4. The system (10) of claim 3, wherein the parallel axes of rotation (A-C) extend in a vertical direction when the user is standing upright.

5. The system (10) of any one of claims 1 to 4, wherein the kinematic chain (26) is a planar revolute kinematic chain.

6. The system (10) of any one of claims 1 to 5, wherein the flexible structure (30) comprises one or more elongate and/or flat elements (30a, 30b) which are configured to introduce a guiding force into the kinematic chain (26) opposite to a direction of tension.

7. The system (10) of claim 6, wherein the one or more elongate and/or flat elements (30a, 30b) are bendable or flexible transverse to the direction of tension

8. The system (10) of any one of claims 1 to 7, wherein the flexible structure (30) comprises elements (30a, 30b) which are connected to one another in an articulated manner.

9. The system (10) of claim 8, wherein an axis of rotation (E) of the elements (30a, 30b) which are connected to one another in an articulated manner extends in a vertical direction when the user stands upright.

10. The system (10) of claim 9, wherein the flexible structure (30) is configured to guide the actuator (24) along a circular path which lies in a horizontal plane when the user stands upright.

11. The system (10) of claim 10, wherein the pivot point of the user's shoulder joint lies at the center of the circular path.

12. The system (10) of any one of claims 8 to 11, wherein one of the elements (30a, 30b) which are connected to one another in an articulated manner comprises a curved section intended to compensate for a vertical displacement of a part of the flexible structure (30) by means of a vertical offset between the ends of the curved section.

13. The system (10) of claim 12, wherein the curved section is L-shaped or U-shaped.

14. A method for reducing forces acting on a user's arm, comprising:
attaching (32) a system (10) of any one of claims 1 to 13 to the user's pelvis and shoulder girdle; and
controlling (34) the actuator (24) to reduce the forces acting on the human's arm;
wherein a hindrance to the lifting of the shoulder resulting from the lifting of a hand of the user is avoided due to the deformability of the flexible structure (30).

## Revendications

1. Système (10) de réduction de forces agissant sur un bras d'un utilisateur, comprenant :
un élément de bassin (12), configuré pour être attaché au bassin de l'utilisateur ; un élément d'épaule (16), configuré pour être attaché à la ceinture scapulaire de l'utilisateur ;
un élément de colonne vertébrale (20), lequel connecte l'élément d'épaule (16) à l'élément de bassin (12) et est configuré pour transférer des forces agissant sur l'élément d'épaule (16) à l'élément de bassin (12) ; et
un agencement (22) pour soutenir le bras de l'utilisateur ;
dans lequel l'agencement (22) présente un actionneur (24), qui est connecté à l'élément d'épaule (16) par une chaîne articulée (26), et la chaîne articulée (26) est configurée pour s'étendre le long de l'omoplate d'une épaule de l'utilisateur et transmettre des forces agissant en direction verticale à l'élément d'épaule (16) ;
**caractérisé en ce que**
la chaîne articulée (26) est guidée au moyen d'une structure flexible (30), laquelle est configurée pour s'étendre au-dessus de l'épaule de l'utilisateur.

2. Système (10) selon la revendication 1, dans lequel la chaîne articulée (26) est configurée pour permettre un mouvement de l'actionneur (24) dans un plan horizontal et empêcher un mouvement de l'actionneur (24) en direction verticale lorsque l'utilisateur se tient debout.

3. Système (10) selon la revendication 2, dans lequel les articulations de la chaîne articulée (26) présentent des axes de rotation parallèles (A-C).

4. Système (10) selon la revendication 3, dans lequel les axes de rotation parallèles (A-C) s'étendent en direction verticale lorsque l'utilisateur se tient debout.

5. Système (10) selon l'une quelconque des revendications 1 à 4, dans lequel la chaîne articulée (26) est conçue en tant que chaîne articulée rotative plane.

6. Système (10) selon l'une quelconque des revendications 1 à 5, dans lequel la structure flexible (30) comprend un ou plusieurs éléments allongés et/ou plats (30a, 30b), qui sont configurés pour transmettre une force de guidage à la chaîne articulée (26) à l'encontre d'une direction de traction.

7. Système (10) selon la revendication 6, dans lequel l'un ou les plusieurs éléments allongés et/ou plats (30a, 30b) sont flexibles ou souples en flexion transversalement à la direction de traction.

8. Système (10) selon l'une quelconque des revendications 1 à 7, dans lequel la structure flexible (30) présente des éléments (30a, 30b) connectés entre eux de manière articulée.

9. Système (10) selon la revendication 8, dans lequel un axe de rotation (E) des éléments (30a, 30b) connectés entre eux de manière articulée s'étend en direction verticale lorsque l'utilisateur se tient debout.

10. Système (10) selon la revendication 9, dans lequel la structure flexible (30) est configurée pour guider l'actionneur (24) sur une trajectoire circulaire dans un plan horizontal lorsque l'utilisateur se tient debout.

11. Système (10) selon la revendication 10, dans lequel le point de rotation de l'articulation d'épaule de l'utilisateur se situe au point central de la trajectoire circulaire.

12. Système (10) selon l'une quelconque des revendications 8 à 11, dans lequel un des éléments connectés entre eux de manière articulée (30a, 30b) présente une section courbée qui est prévue pour compenser un déplacement vertical d'une partie de la structure flexible (30) au moyen d'un décalage vertical entre les extrémités de la section courbée.

13. Système (10) selon la revendication 12, dans lequel la section courbée est en forme de L ou de U.

14. Procédé de réduction de forces agissant sur un bras d'un utilisateur, comprenant :
l'attache (32) d'un système (10) selon l'une quelconque des revendications 1 à 13 au bassin et à la ceinture scapulaire de l'utilisateur ; et
la commande (34) de l'actionneur (24) pour la réduction des forces agissant sur le bras de la personne ;
dans lequel une gêne due au haussement de l'épaule résultant du soulèvement d'une main de l'utilisateur est empêchée par la déformabilité de la structure flexible (30).
